# EUROPEAN PATENT SPECIFICATION

(11) **EP 2 952 229 B1**
(45) Date of publication and mention of the grant of the patent: **25.04.2018**
(21) Application number: 14171627.4
(22) Date of filing: 06.06.2014
(51) Int. Cl.: A61Q 19/00, A61K 8/02, B32B 5/00, D21H 27/00, B05B 13/02

(54) **Apparatus and method for continuously manufacturing moisture film**
Vorrichtung und Verfahren zum kontinuierlichen Herstellen eines Feuchtigkeitsfilms
Appareil et procédé de fabrication en continu de film d'humidité

(43) Date of publication of application: 09.12.2015
(73) Proprietor: Biosol Tech Corporation Limited, New Taipei City 248 (TW)
(72) Inventor: Lin, Yu-Yueh, 114 Taipei City (TW)
(74) Representative: Lang, Christian

(56) References cited:
- EP-A1- 0 309 309
- EP-A1- 2 559 364
- WO-A1-00/72819
- US-A1- 2005 148 261

## Description

### BACKGROUND OF THE INVENTION

The present invention relates to an apparatus and a method for continuously manufacturing moisture films, especially to an apparatus and a method for continuously manufacturing moisture films that include at least one loading surface formed by at least one belt conveyor and used for supporting a lower fabric layer to produce films continuously. The lower fabric layer is moved synchronously with the loading surface and is coated with a first fixing solution layer, a gel layer, and a second fixing solution layer in turn. Thus a film is gradually formed over the lower fabric layer and a continuous manufacturing of moisture films with or without carriers is completed.

The documents US 2005/148261 A1 and WO 00/72819 A1 relate to general prior art on the field of continuously manufacturing film layers onto a substrate, that can be used for facial tissues for cosmetic applications. The US application US 2005/148261 A1 discloses, for example, an apparatus and process for coating a polymeric material onto a nonwoven web for reducing lint and slough levels. The document WO 00/72819 A1 shows another apparatus and process for producing wipes having a functional additive disposed on a tissue substrate for improving the skin cleaning properties of the tissue.

The film-forming materials of the present invention include, but not limited to alginic acid and salt compound of alginic acid (alginate). The alginic acid (so-called gel in the present invention) is a natural polymer, easy to react with salt compounds (such as the first and the second fixing solutions in the present invention, but not limited to) to form alginate film (the film in the present invention). For example, water-soluble sodium alginate (gel) reacts with divalent calcium ions in salt compound solution (fixing solution). Thus crosslinking and curing reactions occur to form insoluble calcium alginate film. During the manufacturing process, the film is produced into a long, continuous/or non-continuous strip with a certain width (but not limited to) and is treated by following processing including cutting masks with required shapes by cutting dies. The masks are applied to cosmetic moisture masks. The so-called film in the present invention is used as facial mask so that the film can also be called as masks. In the present invention, a film with larger area is produced first and then the film is cut into a plurality of masks with certain shaped by cutting dies.

Refer to US 6,080,420, US 6,258,995, US 6,203,845, US 6,201,164, US 6,372,248, US 6,326,524, US 5,144,016, US 5,230,853, US 5,622,666, US 5,660,857, US 5,675,957, US5,144,016, US2005/0287193, US2010/0227164; GB 9419572, GB 9501514, GB 9516930, PCT/GB 9502284(WO96/10106), PCT/GB 9601719(WO97/03710), PCT/ GB 9701098(WO97/39781), PCT/DK 9700292(WO98/ 02196), WO2008/072817, WO03092754, EP2111926A1(WO2008/0090892, PCT/JP2008/050822); JP2010-189386, WO99/20378A1, these are all prior arts related to moisture films (masks). However, most of these prior arts are focused on film composition or weight percent of each component in the film.
Yet there are no technical solutions for problems such as how the moisture films are mass-produced or how the cost is reduced.

Moreover, the moisture film (mask) is divided into two types while in use-without carriers or with carriers. The moisture film without carriers includes only a film with a certain thickness such as alginate mask while the moisture film with carriers consists of a film with a certain thickness and a thin layer of the carrier such as non-woven fabric connected to each other. Generally, the thin layer of the carrier is contained in and integrated with the film so that the film and the carrier are unable or difficult to be separated.

As to the package pattern of the moisture film (mask), the moisture film with or without carriers is folded or directly stored in, but not limited to, a sealed bag with specific solution (such as saline solution for keeping the film moisture or essence/serum for face care). While in use, consumers open the sealed bag and take the moisture film out. The moisture film with or without carriers is a soft and thin membrane. For ease of movement during manufacturing/packaging and convenience in use, a protection film is attached to at least one surface of the moisture film with or without carriers at the manufacturing end. For example, a piece of pearl paper is attached to one surface of the moisture film for supporting during packaging or storage while the other surface of the moisture film is attached with a non-woven fabric layer that allows the solution in the sealed bag to pass and infiltrate into the film. In the present invention, the protection film attached is different from the thin layer of carrier in the moisture film with carriers.

Furthermore, the apparatus or the method for continuously manufacturing moisture films available now has following disadvantage:
First, the moisture film is a soft and thin gel membrane. Thus manufacturing and cutting of the membrane are not so easy. For ease of manufacturing or cutting into shapes required, the inventor proposes an idea that a flat loading surface or a lower fabric layer is used for transporting the moisture film. And the whole film-forming process has been completed during the movement.

Refer to EP2111926A1 (PCT/JP2008/050822, WO2008/0090892), the moisture films with or without carriers are unable to be produced continuously and quickly at the manufacturing end. The manufacturing of the moisture films includes at least coating process of gel (alginic acid) and related device (or working station), coating process of fixing solutions (such as salt compound) and related device (or working station), a device (or working station) in which crosslinking reaction between the gel and the fixing solutions, and/or a device (or working station) for stopping the crosslinking reaction between the fixing solutions and the gel, curing and forming soft moisture films. However, the arrangement of these devices is not efficient. For example, an impregnation coater and a gravure coater are two main workings stations and are separated from each other. Then a continuous supporting material is passed through the above two working stations by turning or traction effect of a plurality of idler wheels or rotating wheels for coating salt compound (fixing solutions) and alginic acid (gel) on the surface of the continuous supporting material. There is no continuous conveyor used to load the supporting material in the whole manufacturing apparatus. Thus each film-forming material such as alginic acid (gel) or salt compound (fixing solutions) is unable to be coated continuously and quickly to form the films. Moreover, several conveys (such as idler wheels or rotating wheels) are required to connect the separated working stations (as shown in figures 2-4 EP2111926A1) at the manufacturing end so that manufacturing of the moisture films can be performed and completed. Thus the whole apparatus and the manufacturing process are more complications and a larger space is occupies. This leads to increasing cost in the apparatus and the manufacturing. And the manufacturing is not a continuous and rapid process.

The third one is that the moisture films are divided into two types-without carriers and with carriers. In prior arts, the same apparatus at the manufacturing end is unable to be used for manufacturing both types of moisture films by simple replacement or modification of devices or working stations in the apparatus. Generally, a new apparatus is designed and used to produce new type of moisture films. This causes a waste, increasing cost and ore space for the new apparatus. The competitiveness is greatly influenced.

There is room for improvement and a need to provide a new design of an apparatus and a method for manufacturing moisture films that overcome above shortcomings.

### SUMMARY OF THE INVENTION

Therefore it is a primary object of the present invention to provide an apparatus for continuously manufacturing moisture films that includes at least one circulating loading surface that is a flat surface facing upward and formed by a belt conveyor. The belt conveyor is moved from an input end (front end) to an output end (rear end) along a conveying direction. A lower fabric layer with water absorbency is arranged at the loading surface and moved backward synchronously with the loading surface to receive various film-forming materials such as fixing solutions and gel for film formation. The following devices are arranged over the loading surface in turn from the input end to the output end. A first fixing solution coating device is used for coating a first fixing solution layer over the lower fabric layer. Then a gel coating device is used to coat a gel layer over the first fixing solution layer for performing crosslinking and curing reactions in an upward direction. A second fixing solution coating device is for coating a second fixing solution layer over the gel layer and carrying out crosslinking and curing reactions in a downward direction. Thereby a film with a certain thickness is gradually formed on a top surface of the lower fabric layer during the synchronous movement of the lower fabric layer with the loading surface and a continuous manufacturing of moisture films without carriers is completed. Therefore both mass production and cost reduction are achieved.

It is another object of the present invention to provide an apparatus for continuously manufacturing moisture films that further includes an upper fabric input device is disposed between the gel coating device and the second fixing solution coating device. An upper fabric layer provided by the upper fabric input device is attached over the gel layer correspondingly. Then the second fixing solution layer is coated over the upper fabric layer so that the upper fabric layer is clipped between the second fixing solution layer and the gel layer after the crosslinking and curing reaction. Thereby a film with a certain thickness and containing the upper fabric layer is gradually formed on a top surface of the lower fabric layer that is moved synchronously with the loading surface. And the original continuous manufacturing of moisture films without carriers is converted to a continuous manufacturing process of moisture films with carriers. Not only mass production and cost reduction are achieved, the types of the moisture film and its convenience of use are also improved.

It is a further object of the present invention to provide an apparatus for continuously manufacturing moisture films that includes a crosslinking control area disposed after the second fixing solution coating device. The moisture film passed through the second fixing solution coating device is washed at the crosslinking control area so that the crosslinking reaction between the fixing solution and the gel in the film is stopped. Thus the flexibility of the film formed can be controlled during manufacturing.

It is a further object of the present invention to provide a method for continuously manufacturing moisture films including following steps. Step 1: provide at least one circulating loading surface used as a working platform for film-forming. The loading surface is a flat surface facing upward and moved circularly along a conveying direction from an input end to an output end. Step 2: provide a lower fabric layer with water absorbency that is moved synchronously with the loading surface for receiving various film-forming materials in turn in and performing film-forming operation. Step 3: provide a first fixing solution coating device that coats a first fixing solution layer over the lower fabric layer. Step 4: provide a gel coating device for coating a gel layer over the first fixing solution layer and having crosslinking and curing reactions in an upward direction therebetween. Step 5: provide a second fixing solution coating device used to coat a second fixing solution layer over the gel layer for carrying out crosslinking and curing reactions mostly in a downward direction. Thus a film with a certain thickness is gradually formed on a top surface of the lower fabric layer moved synchronously with the loading surface. Thereby a continuous manufacturing of the moisture film without carriers is completed.

It is a further object of the present invention to provide a method for continuously manufacturing moisture films that includes following steps. Step 1: provide at least one circulating loading surface used as a working platform for film-forming. The loading surface is a flat surface facing upward and moved circularly along a conveying direction from an input end to an output end. Step 2: provide a lower fabric layer with water absorbency that is moved synchronously with the loading surface for receiving various film-forming materials in turn in and performing film-forming operation. Step 3: provide a first fixing solution coating device used for coating a first fixing solution layer over the lower fabric layer. Step 4: provide a gel coating device for coating a gel layer over the first fixing solution layer and having crosslinking and curing reactions in an upward direction therebetween. Step 5: provide an upper fabric layer attached over the gel layer correspondingly. Step 6: provide a second fixing solution coating device used to coat a second fixing solution layer over the upper fabric layer for carrying out crosslinking and curing reactions mostly in a downward direction. The upper fabric layer is clipped between the second fixing solution layer and the gel layer. Thereby a film with a certain thickness and containing the upper fabric layer is gradually formed on a top surface of the lower fabric layer that is moved synchronously with the loading surface and a continuous manufacturing of the moisture film with carriers is completed.

The lower fabric layer is moveably arranged at the loading surface of the belt conveyor. A roll of the lower fabric layer is disposed in front of the belt conveyor so that the lower fabric layer is passed through the input end and continuously input over the loading surface. Thus the continuous lower fabric layer is moved synchronously with the loading surface to receive various film-forming materials for film formation on the surface thereof. Then the continuous lower fabric layer still supports and moves the film formed to following working stations for follow-up operations.

The lower fabric layer is fixed on the loading surface of the belt conveyor. The lower fabric layer is directly disposed over and moved synchronously with the loading surface of the belt conveyor for receiving various film-forming materials for film formation on the surface thereof. Thus a long strip of the film is formed on the surface of the lower fabric layer over the loading surface of the belt conveyor. After the film-forming process being completed, the lower fabric layer is circularly rotated with the loading surface of the belt conveyor. As to the long strip of the film already formed, it is separated from the lower fabric layer over the loading surface and moved backward to one of follow-up working stations.

A follow-up operation area is disposed after the output end of the circulating loading surface of the belt conveyor. By at least one circulating operation surface that is facing upward, moved along a conveying direction and formed by a belt conveyor, the moisture film without or with carriers already produced is moved backward and entered the follow-up operation area for follow-up processing.

### BRIEF DESCRIPTION OF THE DRAWINGS

Fig. 1 is an embodiment of an apparatus for continuously manufacturing moisture films without carriers according to the present invention;
Fig. 2 is another embodiment of an apparatus for continuously manufacturing moisture films with carriers according to the present invention;
Fig. 3 is a third embodiment of an apparatus for continuously manufacturing moisture films without carriers according to the present invention;
Fig. 4 is a fourth embodiment of an apparatus for continuously manufacturing moisture films with carriers according to the present invention;
Fig. 5 is a fifth embodiment of an apparatus for continuously manufacturing moisture films with carriers according to the present invention;
Fig. 6 is a sixth embodiment of an apparatus for continuously manufacturing moisture films without carriers according to the present invention;
Fig. 7 is a seventh embodiment of an apparatus for continuously manufacturing moisture films without carriers according to the present invention;
Fig. 8 is an eighth embodiment of an apparatus for continuously manufacturing moisture films with carriers according to the present invention;
Fig. 9 is a ninth embodiment of an apparatus for continuously manufacturing moisture films with carriers according to the present invention.

### DETAILED DESCRIPTION OF THE PREFFERED EMBODIMENTS

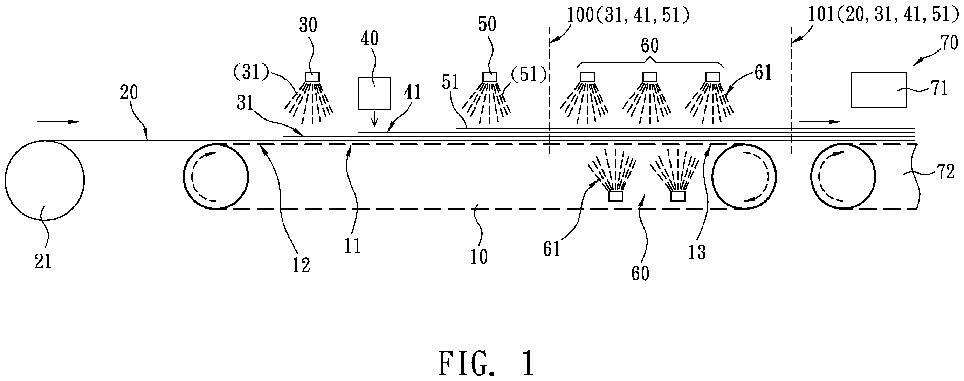

The embodiments of the present invention are shown by side view schematic drawings from Fig. 1 to Fig. 9. The moving direction/conveying direction of an apparatus for continuously manufacturing moisture film of the present invention is from the left side toward the right side from Fig. 1 to Fig. 9. The left side in the figures is defined as the front side while the right side in the figures is defined as the rear side. The direction perpendicular to the surface of the paper with the figures is defined as the width direction. For convenience of description, a lower fabric layer 20, a first fixing solution layer 31, a, gel layer 41, a second fixing solution layer 51 and an upper fabric layer 81 of the embodiments are represented by separated and parallel lines respectively in figures. In fact, the above components 20, 31, 41, 51, 81 are connected to each other closely due to crosslinking reaction therebetween or attached to each other closely due to spray coating, dip coating, etc. There is no gap (as shown in figure) between two adjacent components mentioned above.

Refer to Fig. 1, an apparatus for continuously manufacturing moisture film of the present invention mainly includes at least one belt conveyor 10 with a least one loading surface 11, a lower fabric layer 20 with water absorbency, a first fixing solution coating device 30, a gel coating device 40, and a second fixing solution coating device 50. The three coating devices 30, 40, 50 are arranged over or in front of the loading surface 11 in turn from an input end 12 to an output end 13 along conveying direction of the loading surface 11. The belt conveyor 10 has various forms and the loading surface 11 has different structure according to the form of the belt conveyor 10. The loading surface 11 can be either a continuous surface such as PVC belt conveyor, PU belt conveyor, Teflon belt conveyor, leather belt conveyor, etc., or non-continuous surface such as rolling conveyor, chain conveyor, etc., which is represented by thick broken lines. At least one belt conveyor 10 with a least one loading surface 11 means different combinations of the belt conveyor 10 with the loading surface 11 and there is no limit on the transport way of the belt conveyor 10. The apparatus may include a plurality of belt conveyors 10 each of which has a corresponding loading surface 11. Or a plurality of belt conveyors 10 is connected to form a single loading surface 11. Or a surface of a single belt conveyor 10 is separated into a plurality of segments each of which forms a loading surface 11.

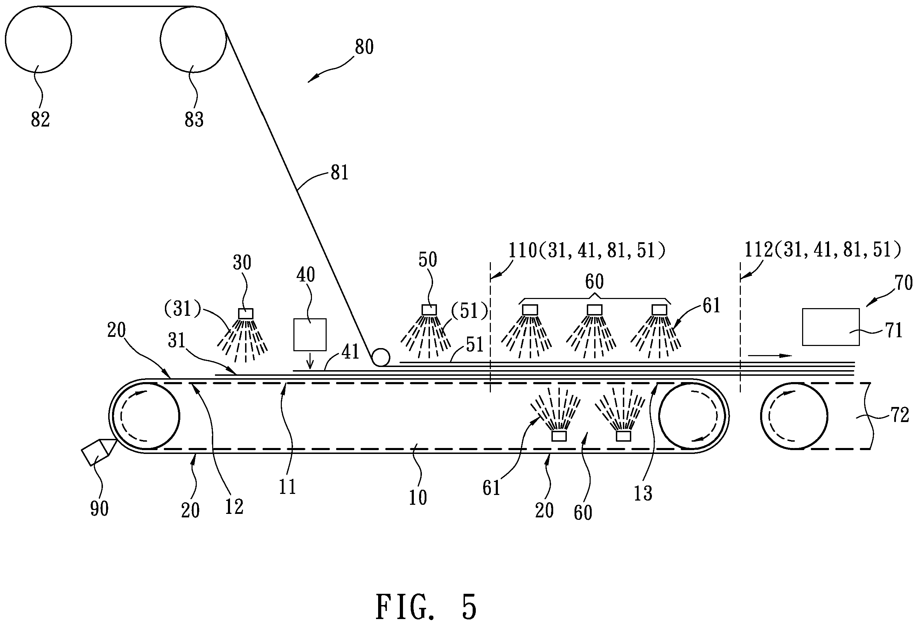

The belt conveyor 10 with at least one loading surface 11 is used as a platform for film-forming, a working surface for continuous formation of moisture films. The circulating loading surface 11 is a flat surface that faces upward and moves along the conveying direction from the input end 12 to the output end 13 circularly. The belt conveyor 10 is designed to have a certain length and a certain width. The loading surface 11 also has a certain length and a certain width so as to form the working surface for forming the moisture film. Moreover, a lower fabric layer 20 with water absorbency is moveably arranged at the loading surface 11 (as shown from Fig. 1 to Fig. 4) or fixed on the loading surface 11 (as shown in Fig. 5). Thus the lower fabric layer 20 is moved synchronously with the loading surface 11. During the synchronous movement, the lower fabric layer 20 is loaded with various film-forming materials such as fixing solutions 31, 51(sodium compound) and gel 41 (alginate), as shown in Fig. 1. Then a film with a certain thickness is gradually and continuously formed on a top surface of the lower fabric layer 20. That means the film-forming is carried out on the lower fabric layer 20.

As shown in Fig. 1, the lower fabric layer 20 is designed into, but not limited to, a continuous long strip of fabric. A supply source 21 is a roll of lower fabric layer 20. The lower fabric layer 20 is provided continuously by the supply source 21 and passed through the input end 12 to be attached to the circulating loading surface 11 smoothly. In Fig. 1, the lower fabric layer 20 and the loading surface 11 are represented by two separate lines. Then the lower fabric layer 20 is moved synchronously with the loading surface 11 and passed through the output end 12 to be output toward the rear side and left the loading surface 11. After being output, the continuous lower fabric layer 20 is delivered into other predetermined follow-up working equipment 70 for following processes such as automatic or laborious film cutting device 71. For example, another belt conveyor 72 is also used together with the film cutting device 71. Thereby a cutting station is formed and is used for cutting continuous moisture film together with the continuous lower fabric layer 20 into a plurality piece of films. Then the films are treated by other working stations such as trimming working station or packaging working station. The follow-up working equipment 70 includes different working stations or other devices according to various requirements for follow-up operations.

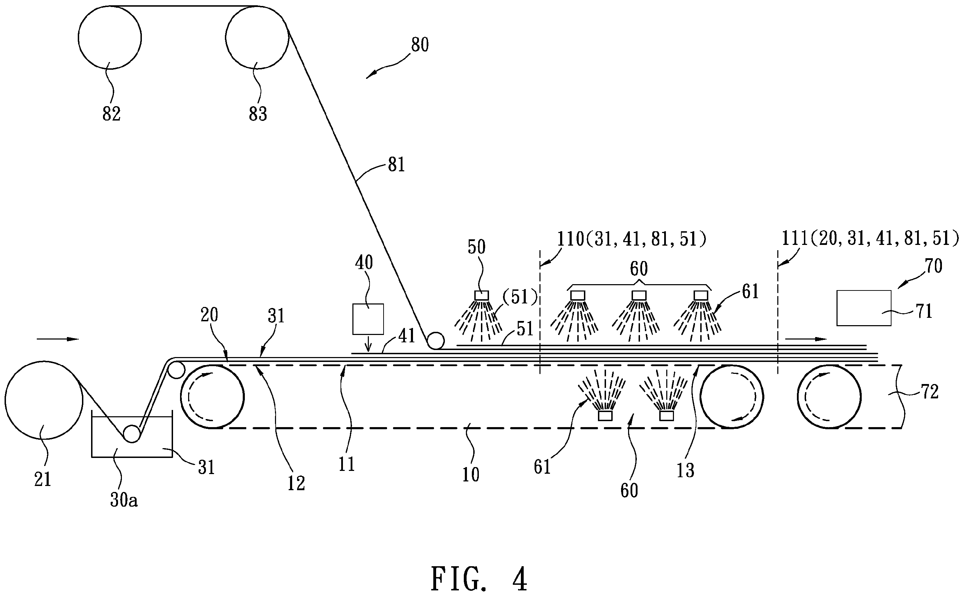

The first fixing solution coating device 30 is used to coat a first fixing solution layer 31 over the lower fabric layer 20 moved together with the loading surface 11. In an embodiment of the present invention, the first fixing solution coating device 30 is arranged over the loading surface 11. As shown in another embodiment of Fig. 4, the first fixing solution coating device 30 can also be disposed in front of the loading surface 11. According to the shape or structure of general moisture film, the first fixing solution layer 31 is evenly spread over the lower fabric layer 20, but not limited. For example, when the moisture film has special structure or shape such as a half-face mask that only covers an upper half or a lower half of user's face, the present invention performs the coating process in an uneven manner. For example, only specific area on the lower fabric layer 20 is coated. Moreover, the way of coating of the present invention is not limited as long as the first fixing solution layer 31 can be formed on the lower fabric layer 20. The way of coating can be spray coating, dip coating or their combination. In Fig. 1, the first fixing solution coating device 30 is coated by spray coating.

As shown in Fig. 1, the gel coating device 40 is arranged at the rear side of the first fixing solution coating device 30. After the first fixing solution layer 31 being coated over the lower fabric layer 20 and moved along with the loading surface 11 to a working range of the gel coating device 40, a gel layer 41 with a certain thickness is coated over the first fixing solution layer 31 by the gel coating device 40. Then the gel layer 41, the first fixing solution layer 31, and the lower fabric layer 20 together with the loading surface 11 are continuously moved backward. During the movement, the first fixing solution layer 31 and the gel layer 41 are crosslinked and gradually cured to form a film.

In this embodiment, the first fixing solution layer 31 is coated over the lower fabric layer 20 first and then the gel layer 41 is coated over the first fixing solution layer 31. Thus the first fixing solution layer 31 is in contact with the lower fabric layer 20 first and may be infiltrate inside through a top surface (or top and bottom surfaces) of the lower fabric layer 20. Yet once a top surface of the first fixing solution layer 31 (containing divalent metal ions) is in contact with the gel layer 41, crosslinking and curing reactions starts immediately. Thus a thin layer of film is formed therebetween. The thin layer of film works like a shielding surface to prevent the gel layer 41 from filtrating inside through the top surface (or bottom surfaces) of the lower fabric layer 20. That means the first fixing solution layer 31 on the surface of the lower fabric layer 20 reacts with the gel layer 41 (from bottom to top) to have crosslinking reaction therebetween and stop infiltrating into the lower fabric layer 20 through the top surface of the lower fabric layer 20 (from top to bottom). Thus a moisture film that is made from hydrogel complex with network structure is formed on the top surface of the lower fabric layer 20. The moisture film (31, 41) formed is easy to be separated from the lower fabric layer 20 while in use. Thus the moisture film of the present invention is considered to have no carrier.

Refer to Fig. 1, the second fixing solution coating device 50 is installed at the rear side of the gel coating device 40. After the gel layer 41 being coated over the first fixing solution layer 31 and moved backward together with the lower fabric layer 20 along the loading surface 11 to a working range of the second fixing solution coating device 50, a second fixing solution layer 51 is coated over the gel layer 41 by the second fixing solution coating device 50. The second fixing solution layer 51, the gel layer 41, the first fixing solution layer 31 and the lower fabric layer 20 are adhered to one another (integrated into one part) and moved backward with the loading surface synchronously after the second fixing solution layer 51 being coated over the gel layer 41. During the movement, the coated second fixing solution layer 51 reacts with the gel layer 41 to initiate crosslinking in the downward direction and gradually curing to form the film. By the crosslinking of the first fixing solution layer 31 with the gel layer 41 and the crosslinking of the second fixing solution layer 51 with the gel layer 41, a film 100 (31, 41, 51) with a certain thickness is formed on the lower fabric layer 20 moved together with the loading surface 11 synchronously to be conveyed backward. Thus the curing period of the film 100 formed over the gel layer 41 is reduced. In other words, the cured film 100 (31, 41, 51) is formed by crosslinking of the first and the second fixing solution layers 31, 51 with the gel layer 41. Thus the cured film 100 in the figure is represented by 31, 41, and 51. Moreover, the film 100 (31, 41, 51) is produced continuously over the lower fabric layer 20 moved backward together with the loading surface 11 synchronously. Thus the film 100 (31, 41, 51) is adhered closely to the lower fabric layer 20 to form a long, continuous strip 101 (20, 31, 41, 51) and moved synchronously with the loading surface 11 to be output through the output end 12 of the loading surface 11. After leaving the loading surface 11, the long, continuous strip 101 (20, 31, 41, 51) is processed by follow-up operations. While leaving the loading surface 11, the film 100 (31, 41, 51) is attached to the lower fabric layer 20 closely to form a long, continuous strip 101 (20, 31, 41, 51). Thus the long, continuous strip 101 in the figure is indicated by the numbers 20, 31, 41, 51. While in use, the film 100 (31, 41, 51) is easily separated from the lower fabric layer 20. Thus a continuous manufacturing of the moisture films without carriers has been completed. The moisture film 100 (31, 41, 51) without carriers is produced rapidly and continuously. Therefore mass production and cost reduction are achieved.

Moreover, the main materials for the first fixing solution layer 31 or the second fixing solution layer 51 include fixing solution required for formation of the film such as salt compound. In practice, the materials for the first fixing solution layer 31 or the second fixing solution layer 51 can also contain other specific ingredient such as essence or serum required for specific functions of the mask such as whitening, anti-wrinkle treatment etc.

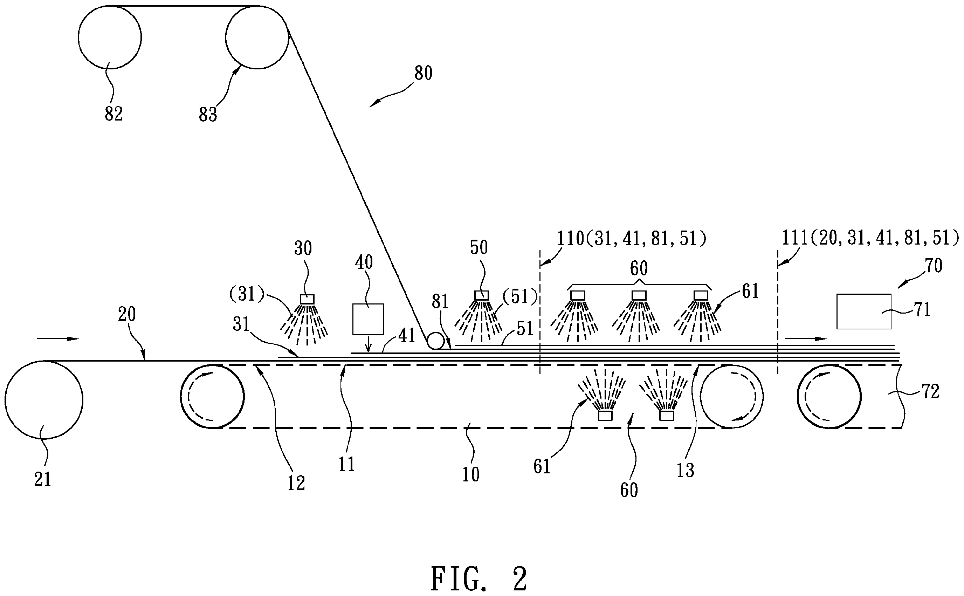

Refer to Fig. 2, another embodiment is revealed. The difference between this embodiment and the above one is in that an upper fabric input device 80 for providing an upper fabric layer 81 is disposed between the gel coating device 40 and the second fixing solution coating device 50. In the figure, the upper fabric layer 81 is flat against the gel layer 41 first. The second fixing solution layer 51 is coated over the upper fabric layer 81 at the same time or later. The upper fabric layer 81 is first in contact with the gel layer 41 so that the gel layer 41 is infiltrated into an inner layer of the upper fabric layer 81 through a bottom surface of the upper fabric layer 81. Thus when the second fixing solution layer 51 is coated over the upper fabric layer 81 that has been infiltrated by the gel layer 41, the second fixing solution layer 51 can be infiltrate into the inner layer of the upper fabric layer 81 through a top surface of the upper fabric layer 81 to react with the gel layer 41 already infiltrated into the inner layer of the upper fabric layer 81 and form crosslinking therebetween. By crosslinking between the first fixing solution layer 31 and the gel layer 41 in the upward direction and crosslinking between the second fixing solution layer 51 and the gel layer 41 in the downward direction, the upper fabric layer 81 is clipped or contained between the second fixing solution layer 51 and the gel layer 41. Thereby during the backward movement of the lower fabric layer 20 with the loading surface 11, a film 110 (31, 41, 81, 51) with a certain thickness and having the upper fabric layer 81 is gradually formed on the top surface of the lower fabric layer 20, as shown in Fig. 2. Thus a continuous manufacturing of the moisture film with carriers has been completed.

In the second embodiment, the upper fabric layer 81 provided by the upper fabric input device 80 is, but not limited to, a long, continuous strip of fabric. The long, continuous strip of the upper fabric layer 81 in Fig. 2 is wound into a roll, used as a supply source 82 for continuous supply of the upper fabric layer 81 and smooth attachment of the upper fabric layer 81 to the gel layer 41. Moreover, the use of mechanical elements such as idle gear 83 or their combinations such as idle gear set can improve convenience in operation or precision of operation of the upper fabric input device 80. Thus the upper fabric layer 81 can be flat attached to the gel layer 41 easily and precisely.

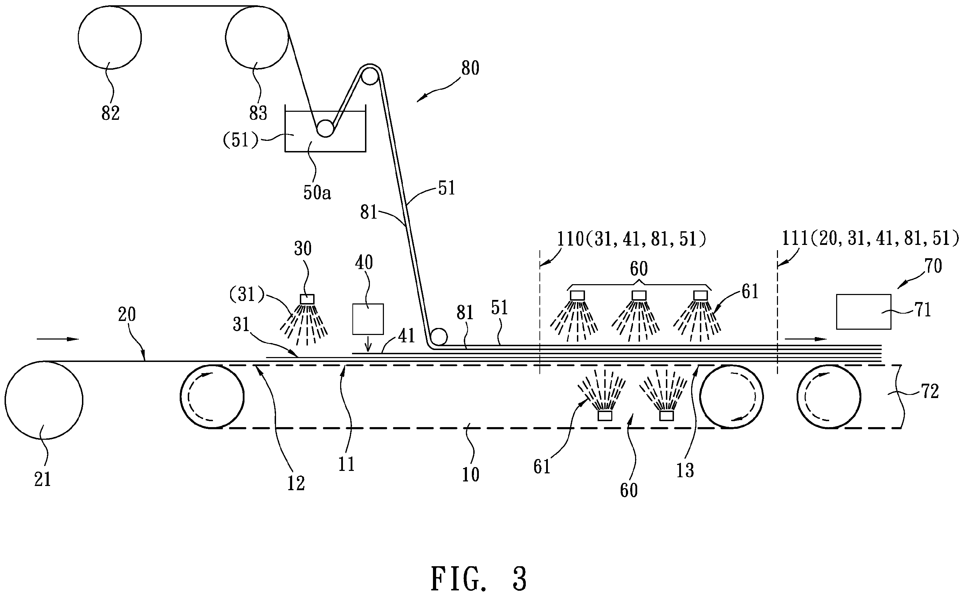

Furthermore, there is no limit on the order of the time point of the second fixing solution layer 51 being coated over the upper fabric layer 81 and the time point of the upper fabric layer 81 being flat attached to the gel layer 41 correspondingly. As shown in Fig. 2, the upper fabric layer 81 is flat attached to the gel layer 41 correspondingly and then the second fixing solution layer 51 is coated over the upper fabric layer 81 by spray coating in the downward direction. Or both of the procedures are carried out at the same time. Thus a continuous manufacturing of the moisture film with carriers has been completed. Refer to Fig. 3, the second fixing solution layer 51 is coated over the upper fabric layer 81 by dip coating. Then the upper fabric layer 81 coated with the second fixing solution layer 51 is attached to the gel layer 41 smoothly and correspondingly. Due to the second fixing solution layer 51 existed between the upper fabric layer 81 and the gel layer 41, the upper fabric layer 81 in Fig. 3 can be easily separated from the film 110 (31, 41, 81, 51) formed. Thus a continuous manufacturing of the moisture film without carriers as shown in Fig. 3 is revealed.

In Fig. 2, the film 110 is formed by crosslinking between the first fixing solution layer 31 and the gel layer 41 in the upward direction as well as crosslinking between the second fixing solution layer 51 and the gel layer 41 in the downward direction and through the upper fabric layer 81. Thus the upper fabric layer 81 in the film 110 (31, 41, 81, 51) formed is unable to be separated from the film 110 (31, 41, 81, 51) easily. Compared the second embodiment in Fig. 2 with the first embodiment in Fig. 1, the film 110 (31, 41, 81, 51) of the second embodiment including the upper fabric layer 81 integrated therewith is formed continuously on the lower fabric layer 20 that is moved backward with the loading surface 11 synchronously. In practice, the film 110 (31, 41, 81, 51) and the lower fabric layer 20 are adhered to form a long, continuous strip 111 (20, 31, 41, 81, 51). As shown in Fig. 2, the long, continuous strip 111 (20, 31, 41, 81, 51) is moved synchronously with the loading surface 11 to be output through the output end 12 of the loading surface 11. After leaving the loading surface 11, the long, continuous strip 111 (20, 31, 41, 81, 51) is processed by follow-up operations. During follow-up operations, the film 110 (31, 41, 81, 51) and the lower fabric layer 20 are still adhered to each other to form the long, continuous strip 111 (20, 31, 41, 81, 51). While in use, the film 110 (31, 41, 81, 51) can be easily separated from the lower fabric layer 20 after being cut into various shapes of masks ready to use. Yet the film 110 (31, 41, 81, 51) of the second embodiment includes the upper fabric layer 81. Thus the film 110 (31, 41, 81, 51) of the second embodiment is considered as the moisture film with carriers in the present invention. Therefore the second embodiment reveals a continuous manufacturing of the moisture film with carriers. The moisture film 110 (31, 41, 81, 51) with carriers is produced rapidly and continuously so as to achieve mass production and cost reduction.

Moreover, a continuous manufacturing of moisture films without carriers shown in Fig. 1 (the first embodiment) is converted into a continuous manufacturing of moisture films with carriers shown in Fig. 2 (the second embodiment) by the arrangement of the upper fabric input device 80 and disposition of the upper fabric layer 81 provided by the upper fabric input device 80. Thus manufacturers can determine to produce the moisture films with carriers or without carriers easily and conveniently according to consumers' requirements. Thus not only mass production and cost reduction are achieved, consumers also have more options of moisture films and convenience of use.

Refer to Fig. 1 and Fig. 2, a crosslinking control area 60 is disposed on the rear side of the second fixing solution coating device 50. The crosslinking control area 60 includes, but not limited to, at least one crosslinking control device. From Fig. 1 to Fig. 5, the crosslinking control area 60 includes three and two spray-type crosslinking control devices respectively arranged at the top surface and the bottom surface of the loading surface 11 (or the lower fabric layer 20), but not limited to. The crosslinking control area 60 is used for washing the film 100 (31, 41, 51) formed in Fig. 1 or the film 110 (31, 41, 81, 51) shown in Fig. 2. The crosslinking reaction between the fixing solution layers 31, 51 and the gel layer 41 is stopped by the washing process provided in the crosslinking control area 60. For example, a part of the fixing solution 31, 51 not reacted yet is washed out by pure water. Or the crosslinking reaction between the fixing solution layers 31, 51 and the gel layer 41 is stopped directly by crosslinking stop solution. Thus the crosslinking and curing reactions between the first and second fixing solution layers 31, 51 and the gel layer 41 can be controlled or stopped. By controlling washing period or travel distance of the crosslinking control area 60, a part of the fixing solutions in the fixing solution layers 31, 51 is washed out so as to reduce reaction time and degree of the crosslinking and curing reactions between the first and second fixing solution layers 31, 51 and the gel layer 41 and avoid over-curing. Thereby the film 100 (31, 41, 51) or the film 110 (31, 41, 81, 51) formed is more flexible.

In addition, crosslinking control solution 61 of the crosslinking control area 60 can be either water used for washing, or crosslinking stop solution for stopping the crosslinking reaction between the fixing solution layers 31, 51 and the gel layer 41. In practice, the crosslinking control solution 61 can also be added with specific ingredients such as cosmetic essence/serum according to additional functions such as whitening, anti-wrinkle treatment, etc. the mask requires.

Refer to Fig. 3, a further embodiment is disclosed. The difference between this embodiment and the above one in Fig. 2 is in that the coating way of the second fixing solution coating device 50 in Fig. 2 is changed into a dip coating carried out by a dip-type second fixing solution coating device 50a. Thereby an upper fabric layer 81 provided by an upper fabric input device 80 is first passed through the dip-type second fixing solution coating device 50a. As shown in Fig. 3, then a second fixing solution layer 51 represented by a parallel line is coated over the upper fabric layer 81. Yet the second fixing solution layer 51 can be, but not limited to, coated over one surface of the upper fabric layer 81. In this embodiment, the upper fabric layer 81 can be easily removed from the film 110 (31, 41, 81, 51). A continuous manufacturing of moisture films without carriers is shown in Fig. 3.

Refer to Fig. 4, the difference of the embodiment in the figure and the second embodiment shown in Fig. 2 is in that the first fixing solution coating device 30 of the second embodiment is replaced by an equivalent dip-type first fixing solution coating device 30a. Thereby the lower fabric layer 20 provided by a lower fabric input device 21 is first passed through the dip-type first fixing solution coating device 30a. As shown in Fig. 4, then a first fixing solution layer 31 represented by a parallel line is coated over the lower fabric layer 20. Yet the first fixing solution layer 31 is not limited to be coated over the top surface of the lower fabric layer 20. Thereby the first fixing solution coating device 30a of the embodiment achieves the same use and function as the first fixing solution coating device 30 in the second embodiment shown in Fig. 2 (or the first embodiment in Fig. 1). That means the first fixing solution layer 31 is coated over the lower fabric layer 20 which is moved backward synchronously with the loading surface 11 by the first fixing solution coating device 30a.

Refer to Fig. 5, a further embodiment is revealed. The difference between the fifth embodiment and the above four embodiments is in that the lower fabric layer 20 of this embodiment with water absorbency is fixed on and moved backward synchronously with the loading surface 11 while the lower fabric layer 20 with water absorbency of the above embodiments is moveably arranged at and moved backward synchronously with the loading surface 11. Due to that the lower fabric layer 20 is fixed on the surface of the belt conveyor 10, the lower fabric layer 20 is moved and rotated synchronously with the belt conveyor 10. Thus the lower fabric layer 20 forms the circulating loading surface 11 shown in Fig. 1 to Fig. 4 that is used as a working surface for continuous formation of the moisture film when the lower fabric layer 20 is rotated to the top surface of the belt conveyor 10 along with the belt conveyor 10. The use and function of the lower fabric layer 20 can still be achieved. In practice, the film 110 (31, 41, 81, 51) in the fifth embodiment is attached to the lower fabric layer 20 to be moved backward synchronously as long as the film 110 (31, 41, 81, 51) still stays on and moved backward synchronously with the loading surface 11, no matter the film (31, 41, 81, 51) is having crosslinking and curing reactions, already formed or being washed by the crosslinking control area 60. When the film 110 (31, 41, 81, 51) is output through the output end 12 of the loading surface 11, the film 110 (31, 41, 81, 51) is separated from the lower fabric layer 20 to form a long, continuous strip 112 (31, 41, 81, 51) for follow-up operations, as shown in Fig. 5. The long, continuous strip 112 (31, 41, 81, 51) output through the output end 12 of the loading surface 11 in the fifth embodiment is different from the long, continuous strip 111 (20, 31, 41, 81, 51) in the embodiments 2, 3 and 4 because that the lower fabric layer 20 of the fifth embodiment is fixed on the loading surface 11, not being moved backward with the long, continuous strip 112 (31, 41, 81, 51) to be processed by follow-up operations.

In the fifth embodiment, the lower fabric layer 20 fixed on the surface of the belt conveyor 10 is rotated circularly and synchronously with the belt conveyor 10 and all coating processes are carried out under an open state. Thus there may be a part of film-forming material such as fixing solutions, 21, 51, gel 41 or film 110 already formed residual on the circulating loading surface 11 and/or the lower fabric layer 20. This may result in damages on the film 110 (31, 41, 81, 51) formed in the next cycle. Thus a scraping and cleaning tool 90 such as a scraper is arranged at the apparatus. When the lower fabric layer 20 or the circulating loading surface 11 continues to rotate and form the loading surface 11 of the next cycle, the residues on the circulating loading surface 11 or the lower fabric layer 20 are removed or cleaned up by the scraping and cleaning tool 90 so as to prevent the quality of the film 110 (31, 41, 81, 51) from being affected by the residues.

Moreover, the fourth and the fifth embodiments of the present invention are modified by and equivalent to the second embodiment that is an apparatus/or method for continuous manufacturing of a moisture film with carriers. But these two embodiments are not limited to be equivalent to the second embodiment. They can also be equivalent to the first embodiment that is an apparatus/or method for continuous manufacturing of a moisture film without carriers.

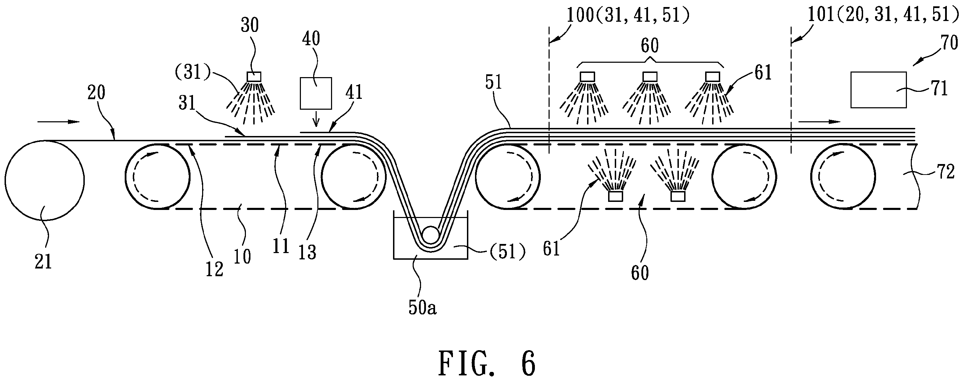

Refer to Fig. 6, a further embodiment is disclosed. The difference between this (the sixth) embodiment and the first embodiment shown in Fig. 1 is in that the spray-type second fixing solution coating device 50 of the first embodiment is replaced by an equivalent dip-type second fixing solution coating device 50a. Thereby the assembly 20, 31, 41 of the first fixing solution layer 31, the gel layer 41 and the lower fabric layer 20 formed after the gel layer 41 being coated over the first fixing solution layer 31 is continuously moved backward to pass through the dip-type second fixing solution coating device 50a. Then the gel layer 41 is added with a second fixing solution layer 51 represented by a parallel line, but the second fixing solution layer 51 is not limited to be only dip-coated over the top surface of the gel layer 41. The second fixing solution coating device 50a of this embodiment provides the same use and function as the second fixing solution coating device 50 of the first embodiment in Fig. 1. That means a second fixing solution layer 51 is coated over the assembly 20, 31, 41 moved backward by the dip-type second fixing solution coating device 50a.

Furthermore, in the sixth embodiment, the loading surface 11 can be considered as a two-stage loading surface 11, disposed in front of and at the rear end of the dip-type second fixing solution coating device 50a respectively. Or the loading surface 11 is considered as two separated loading surfaces 11 each of which is formed by a belt conveyor 10.

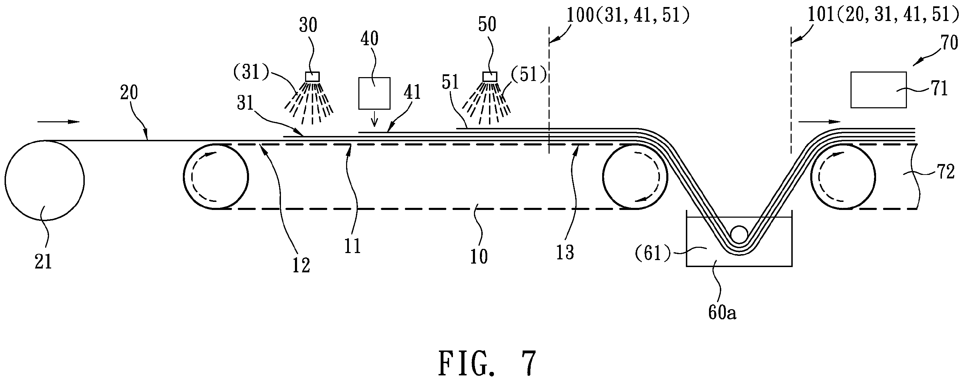

As shown in Fig. 7, a seventh embodiment is disclosed. The difference between this (the seventh) embodiment and the first embodiment shown in Fig. 1 is in that the spray-type crosslinking control area 60 of the first embodiment is replaced by an equivalent dip-type crosslinking control area 60a. When the film 100 (31, 41, 51) and the lower fabric layer 20 have been connected to form an assembly (20, 31, 41, 51) and the assembly (20, 31, 41, 51) has been moved backward with the loading surface 11 synchronously to be output and passed through the dip-type crosslinking control area 60a, crosslinking control solution 61 of the crosslinking control area 60 is used to wash the fixing solution layer residual in the film 100 (31, 41, 51) and/or the gel layer 41, or directly stopping the crosslinking reaction between the fixing solution layers 31, 51 and the gel layer 41. Thus the dip-type crosslinking control area 60a has the same use and function as the spray-type crosslinking control area 60 of the first embodiment.

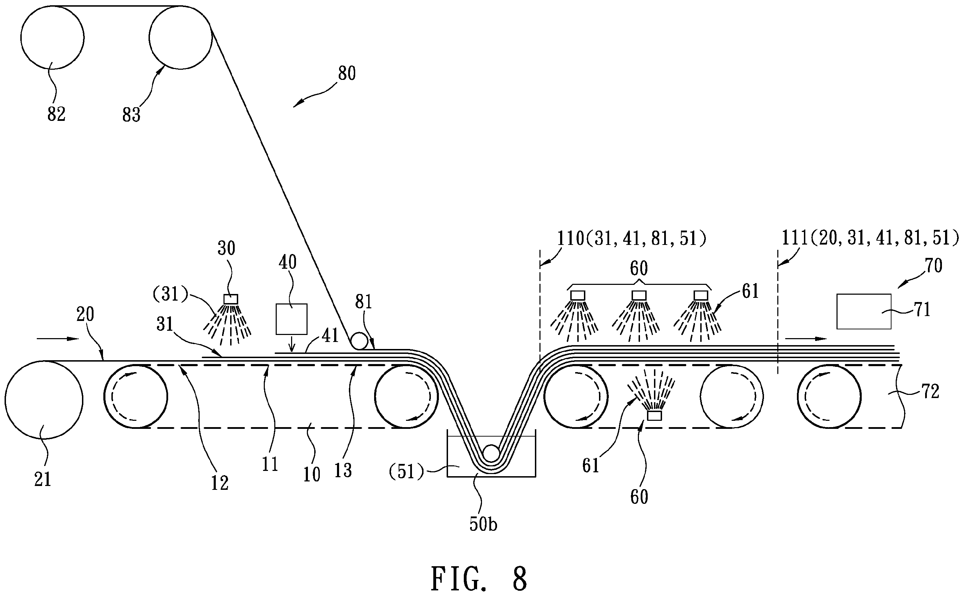

Refer to Fig. 8, a further embodiment is revealed. The difference between this (the eighth) embodiment and the second embodiment shown in Fig. 2 is in that the spray-type second fixing solution coating device 50 of the second embodiment is replaced by an equivalent dip-type second fixing solution coating device 50b. After the gel layer 41 has been coated over the first fixing solution layer 31 and the upper fabric layer 81 has been attached to the gel layer 41 smoothly, the assembly 20, 31, 41, 81 formed by the first fixing solution layer 31, the gel layer 41, the upper fabric layer 81 and the lower fabric layer 20 is moved backed with the loading surface 11 synchronously and continuously to pass through the dip-type second fixing solution coating device 50b. Then as shown in Fig. 8, a second fixing solution layer 51 represented by a parallel line is added over the gel layer 41. Yet the second fixing solution layer 51 is not limited to be added only over the top surface of the gel layer 41. Thereby the dip-type second fixing solution coating device 50b of this embodiment has the same use and function as the second fixing solution coating device 50 of the second embodiment shown in Fig. 2 (or the second fixing solution coating device 50a of the sixth embodiment). That means a second fixing solution layer 51 is coated over the assembly (20, 31, 41, 81) moved backward by the dip-type second fixing solution coating device 50b. Moreover, the structure of the loading surface 11 in this embodiment is similar to the two-stage loading surface 11 or two separated loading surfaces 11 of the sixth embodiment.

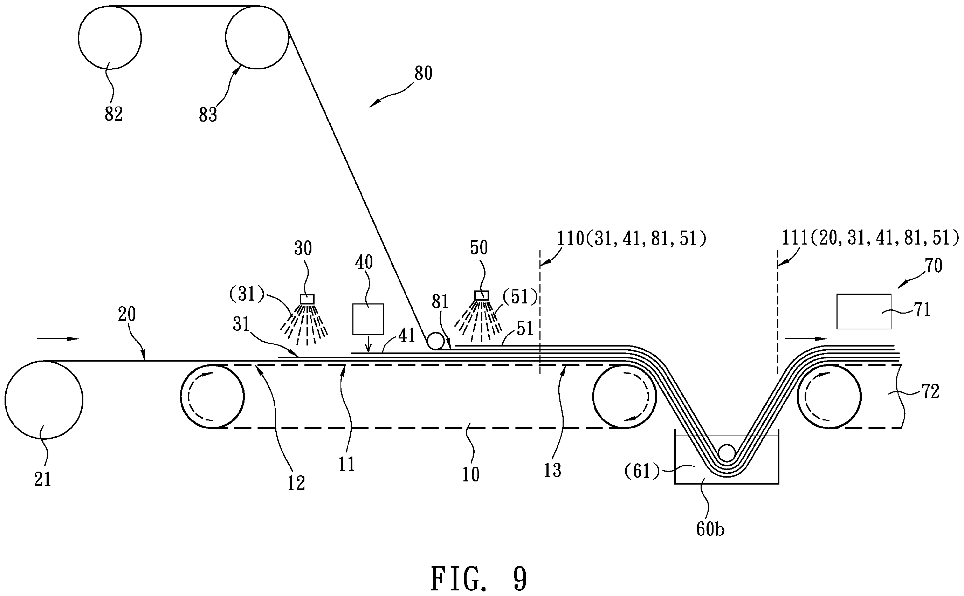

Refer to Fig. 9, a ninth embodiment is disclosed. The difference between this embodiment and the second embodiment shown is in that the spray-type crosslinking control area 60 of the second embodiment is replaced by an equivalent dip-type crosslinking control area 60b. When the film 110 (31, 41, 81, 51) and the lower fabric layer 20 has been moved backward with the loading surface 11 synchronously to be output, the film 110 (31, 41, 81, 51) is continuously delivered backward to pass through the dip-type crosslinking control area 60b. In the dip-type crosslinking control area 60b shown in Fig. 9, crosslinking control solution 61 is used to wash the fixing solution layers 31, 51 residual in the film 110 (31, 41, 81, 51), or the gel layer 41, or directly stopping the crosslinking reaction between the fixing solution layers 31, 51 and the gel layer 41. Thus this embodiment has the same function and purpose as the spray-type crosslinking control area 60 of second embodiment or the dip-type crosslinking control area 60a of the seventh embodiment.

By means of the apparatus for continuously manufacturing moisture films mentioned above, the present invention provides a method for continuously manufacturing moisture films without carriers that includes following steps:
Step 1: provide at least one circulating loading surface used as a working platform for film-forming. The loading surface is a flat surface facing upward and moved circularly along a conveying direction from an input end to an output end.
Step 2: provide a lower fabric layer with water absorbency that is moved synchronously with the loading surface for receiving various film-forming materials in turn and performing film-forming operation.
Step 3: provide a first fixing solution coating device that coats a first fixing solution layer over the lower fabric layer by spray coating or dip coating.
Step 4: provide a gel coating device for coating a gel layer over the first fixing solution layer and having crosslinking and curing reactions between the gel layer and the first fixing solution layer.
Step 5: provide a second fixing solution coating device used to coat a second fixing solution layer over the gel layer for carrying out crosslinking and curing reactions between the second fixing solution layer and the gel layer. The second fixing solution layer is coated over the gel layer by spray coating or dip coating. Thus a film with a certain thickness is gradually formed on a top surface of the lower fabric layer moved synchronously with the loading surface. Therefore a continuous manufacturing of the moisture film without carriers is completed.

Moreover, the method further includes a step 6 after the step 5-provide a crosslinking control area that controls crosslinking reaction in the film gradually formed on the lower fabric layer. By at least one crosslinking stop solution that is distributed by spray coating or dip coating in the crosslinking control area, the crosslinking reaction between the first fixing solution layer and the gel layer and/or the crosslinking reaction between the second fixing solution layer and the gel layer is stopped or washed out.

By means of the apparatus for continuously manufacturing moisture films mentioned above, the present invention provides a method for continuously manufacturing moisture films with carriers that includes following steps:
Step 1: provide at least one circulating loading surface used as a working platform for film-forming. The loading surface is a flat surface facing upward and moved circularly along a conveying direction from an input end to an output end.
Step 2: provide a lower fabric layer with water absorbency that is moved synchronously with the loading surface for receiving various film-forming materials in turn and performing film-forming operation.
Step 3: provide a first fixing solution coating device that coats a first fixing solution layer over the lower fabric layer by spray coating or dip coating.
Step 4: provide a gel coating device for coating a gel layer over the first fixing solution layer and having crosslinking and curing reactions between the gel layer and the first fixing solution layer.
Step 5: provide an upper fabric layer attached over the gel layer correspondingly.
Step 6: provide a second fixing solution coating device used to coat a second fixing solution layer over the upper fabric layer by spray coating or dip coating for carrying out crosslinking and curing reactions between the second fixing solution layer and the gel layer; thereby the upper fabric layer is clipped between the second fixing solution layer and the gel layer due to the crosslinking reaction therebetween; a film with a certain thickness and containing the upper fabric layer is gradually formed on a top surface of the lower fabric layer that is moved synchronously with the loading surface and a continuous manufacturing of the moisture film with carriers is completed.

Furthermore, the method includes a step 7 after the above step 6. Provide a crosslinking control area used for control of crosslinking reaction in the film gradually formed on the lower fabric layer. At least one crosslinking control solution of the crosslinking control area is used for washing and stopping the crosslinking reaction between the second fixing solution layer and the gel layer and/or the second fixing solution layer and the gel layer. The crosslinking control solution is sprayed over the film or the film is dipped therein.

Additional advantages and modifications will readily occur to those skilled in the art. Therefore, the invention in its broader aspects is not limited to the specific details, and representative devices shown and described herein. Accordingly, various modifications may be made without departing from the general inventive concept as defined by the appended claims.

## Claims

1. An apparatus suitable for continuously manufacturing films (100), comprising: at least one belt conveyor (10), a lower fabric layer (20), and coating devices (30, 40, 50)
**characterized in that**
said coating devices comprise three coating devices (30, 40, 50) suitable for, in use, coating a first, second and third compound (31, 41,51) in turn onto the lower fabric layer (20) and
said belt conveyor (10) includes at least one circulating loading surface (11) used as a working platform for forming the film (100, 110) and the circulating loading surface (11) includes a flat surface facing upward and is suitable for, in use, circular movement along a conveying direction from an input end (12) to an output end (13) thereof; said loading surface is adapted for disposing the lower fabric layer (20) on the surface of the loading surface (11) and, in use, can be moved synchronously with the loading surface (11) so as to receive various film-forming materials (31, 41, 51) in turn during movement of the lower fabric layer (20) for gradually and continuously forming the film (100, 110);
wherein the three coating devices (30, 40, 50) are arranged over or in front of the loading surface (11) in turn from an input end (12) to an output end (13) along a conveying direction of the loading surface (11); and **in that** the film is a moisture film (100) for cosmetic facial moisture masks;
the lower fabric layer (20) has water absorbency;
the coating devices are provided as a first fixing solution coating device (30) suitable for, in use, coating a first fixing solution layer (31), a gel coating device (40) suitable for, in use, coating a gel layer (41), and a second fixing solution coating device (50) suitable for, in use, coating a second fixing solution layer (51);
wherein the gel coating device (40) is disposed after the first fixing solution coating device (30) and the second fixing solution coating device (50) is disposed after the gel coating device (40);
wherein the gel coating device (40) is configured to provide a film-forming material,
preferably including alginic acid and salt compounds of alginic acid (alginate); and
wherein the first and the second fixing solutions to be applied by the first fixing solution coating device (30) and the second fixing solution coating device (50) include solutions for curing and fixing the water-soluble film-forming agent of the gel layer (41) to form an insoluble gel membrane film.

2. The apparatus as claimed in claim 1, wherein an upper fabric input device (80) for providing an upper fabric layer (81) is disposed between the gel coating device (40) and the second fixing solution coating device (50), so that the upper fabric input device (80) is suitable, in use, for firstly attaching the upper fabric layer (81) over the gel layer (41) correspondingly, wherein the upper fabric layer (81) is suitable for being coated by the second fixing solution layer (51).

3. The apparatus as claimed in claim 1 or claim 2, wherein the first fixing solution coating device (30) is suitable for, in use, coating the first fixing solution layer (31) by spray coating, dip coating, or their combination.

4. The apparatus as claimed in claim 1 or claim 2, wherein the second fixing solution coating device (50) is suitable for, in use, coating the second fixing solution layer (51) by spray coating, dip coating, or their combination.

5. The apparatus as claimed in claim 1 or claim 2, wherein materials for the first fixing solution layer (31) and the second fixing solution layer (51) only include solutions for curing and fixing, preferably including salt compounds reactive with the water-soluble film-forming agent of the gel layer (41), or include a combination of the solution for curing and fixing with specific ingredients for providing specific functions to the moisture films (100, 110).

6. The apparatus as claimed in claim 1 or claim 2, wherein a crosslinking control area (60) is disposed after the second fixing solution coating device (50) and is suitable for, in use, distributing at least one crosslinking control solution (61) by spray coating or dip coating in the crosslinking control area (60) to control of the crosslinking reaction in the moisture film (100, 110) gradually formed on the lower fabric layer (20); so that the crosslinking between the first fixing solution layer (31) and the gel layer (41) and/or the crosslinking between the second fixing solution layer (51) and the gel layer (41) is washed out or stopped.

7. The apparatus as claimed in claim 6, wherein the crosslinking control solution (61) distributed in the crosslinking control area (60) includes pure water, crosslinking stop solution, pure water with specific ingredients providing specific functions to the moisture films (100, 110) and crosslinking stop solution with specific ingredients providing specific functions to the moisture films (100, 110).

8. The apparatus as claimed in claim 2, wherein the upper fabric layer (81) provided by the upper fabric input device (80) is a continuous long strip of fabric provided continuously and attached over the gel layer (41) correspondingly.

9. The apparatus as claimed in claim 1 or claim 2, wherein the lower fabric layer (20) is a continuous long strip of fabric entered the circulating loading surface through the input end (12) of the circulating loading surface (11), attached to the loading surface smoothly and moved synchronously with the loading surface (11).

10. The apparatus as claimed in claim 1 or claim 2, wherein the lower fabric layer (20) is fixed on the circulating loading surface (11) of the belt conveyor (10) so that the lower fabric layer (20) is rotated synchronously and circularly with the belt conveyor (10).

11. The apparatus as claimed in claim 10, wherein a scraping and cleaning tool (90) is arranged at a surface of the lower fabric layer (20) rotated synchronously and circularly with the belt conveyor (10) and the scraping and cleaning tool (90) is used for scraping and cleaning residual fixing solution (31, 51) or gel (41) of the circulating loading surface (11) or the lower fabric layer (20) after formation of the moisture film (100, 110).

12. A method for continuously manufacturing moisture films performed by the apparatus claimed in one of the claims ranging from claim 1 to claim 11 comprising the steps of:
Step 1: providing at least one circulating loading surface (11) used as a working platform for film-forming and the loading surface (11) including a flat surface facing upward and moved circularly along a conveying direction from an input end (20) to an output end (13);
Step 2. providing a lower fabric layer (20) with water absorbency that is moved synchronously with the loading surface (11) for receiving various film-forming materials (31, 41, 51) in turn in and performing film-forming operation;
Step 3: coating a first fixing solution layer (31) over the lower fabric layer (20) by a first fixing solution coating device (30) by spray coating or dip coating while the lower fabric layer (20) is moved synchronously with the loading surface (11);
Step 4: coating a gel layer (41) over the first fixing solution layer (31) by the gel coating device (40) while the lower fabric layer (20) is moved backward synchronously with the loading surface (11) to a working range of the gel coating device (40) and having crosslinking and curing reactions between the gel layer (41) and the first fixing solution layer (31); and
Step 5: coating a second fixing solution layer (51) the gel layer (41) by the second fixing solution coating device (50) by spray coating or dip coating while the gel layer (41) being coated over the first fixing solution layer (31) is moved backward synchronously with the loading surface (11) to a working range of the second fixing solution coating device (50), wherein by the crosslinking and curing reactions between the second fixing solution layer (51) and the gel layer (41) a moisture film (100) with a certain thickness is gradually formed on a top surface of the lower fabric layer (20) that is moved synchronously with the loading surface and a continuous manufacturing of the moisture films (100) without carriers is completed.

13. The method as claimed in claim 12, wherein after the step 5, the method further includes a step 6 of providing a crosslinking control area (60) that controls crosslinking reaction in the moisture film (100) gradually formed on the lower fabric layer (20); by at least one crosslinking stop solution that is distributed by spray coating or dip coating in the crosslinking control area (60), the crosslinking reaction between the first fixing solution layer (31) and the gel layer (41) and the crosslinking reaction between the second fixing solution layer (51) and the gel layer (41) are stopped or washed out.

14. A method for continuously manufacturing moisture films performed by the apparatus claimed in one of the claims ranging from claim 1 to claim 11 comprising the steps of:
Step 1: providing at least one circulating loading surface (11) used as a working platform for film-forming and the loading surface including a flat surface facing upward and moved circularly along a conveying direction from an input end (12) to an output end (13);
Step 2: providing a lower fabric layer (20) with water absorbency that is moved synchronously with the loading surface (11) for receiving various film-forming materials (31, 41, 51) in turn in and performing film-forming operation;
Step 3: coating a first fixing solution layer (31) over the lower fabric layer (20) by the first fixing solution coating device (30) by means of spray coating or dip coating;
Step 4: coating a gel layer (41) over the first fixing solution layer (31) by the gel coating device (40) and having crosslinking and curing reactions between the gel layer (41) and the first fixing solution layer (31); and
Step 5: providing an upper fabric layer (81) attached over the gel layer (41) correspondingly;
Step 6: coating a second fixing solution layer (51) over the upper fabric layer (81) by spray coating or dip coating by the second fixing solution coating device (50) to initiate crosslinking and curing reactions between the second fixing solution layer (81) and the gel layer (41); thereby the upper fabric layer (81) is clipped and contained between the second fixing solution layer (51) and the gel layer (41) due to the crosslinking reaction there between; wherein a moisture film (110) with a certain thickness and containing the upper fabric layer (20) is gradually formed on a top surface of the lower fabric layer that is moved synchronously with the loading surface and a continuous manufacturing of the moisture films (110) with carriers is completed.

15. The method as claimed in claim 14, wherein after the step 6, the method further includes a step 7 of providing a crosslinking control area (60) that controls crosslinking reaction in the moisture film (110) gradually formed on the lower fabric layer (20); by at least one crosslinking stop solution that is distributed by spray coating or dip coating in the crosslinking control area (60), the crosslinking reaction between the first fixing solution layer (31) and the gel layer (41) and the crosslinking reaction between the second fixing solution layer (51) and the gel layer (41) are stopped or washed out.

## Patentansprüche

1. Zur kontinuierlichen Herstellung von Filmen (100) geeignete Apparatur, welche zumindest einen Bandförderer (10), eine untere Stofflage (20) und Beschichtungsvorrichtungen (30, 40, 50) umfasst,
**dadurch gekennzeichnet, dass**
die Beschichtungsvorrichtungen drei Beschichtungsvorrichtungen (30, 40, 50) umfassen, welche sich im Gebrauch dazu eignen, um eine erste, zweite und dritte Zusammensetzung (31, 41, 51) der Reihe nach auf die untere Stofflage (20) aufzubringen,
wobei der Bandförderer (10) zumindest eine umlaufende Beladungsfläche (11) aufweist, welche als eine Arbeitsplattform zur Bildung des Films (100, 110) verwendet wird, wobei die umlaufende Beladungsfläche (11) eine nach oben weisende flache Oberfläche beinhaltet und im Gebrauch eine kreisförmige Bewegung entlang einer Förderrichtung von einem Eingangsende (12) zu einem Ausgangsende (13) davon ausführen kann,
wobei die Beladungsfläche so angepasst ist, dass die untere Stofflage (20) auf der Oberfläche der Beladungsfläche (11) angeordnet werden kann und im Gebrauch mit der Beladungsfläche (11) synchron bewegt werden kann, sodass während der Bewegung der unteren Stofflage (20) der Reihe nach verschiedene filmbildende Materialien (31, 41, 51) aufgenommen werden können, um allmählich und kontinuierlich den Film (100, 110) auszubilden,
wobei die drei Beschichtungsvorrichtungen (30, 40, 50) der Reihe nach von einem Eingangsende (12) zu einem Ausgangsende (13) entlang einer Förderrichtung der Beladungsfläche (11) über oder vor der Beladungsfläche (11) angeordnet sind, und dass
der Film ein Feuchtigkeitsfilm (100) für kosmetische Gesichtsfeuchtigkeitsmasken ist,
die untere Stofflage (20) wasseraufnahmefähig ist,
wobei die Beschichtungsvorrichtungen als eine erste Fixierlösungsbeschichtungsvorrichtung (30), die im Gebrauch dazu dient, um eine erste Fixierlösungsschicht (31) aufzutragen, eine Gelbeschichtungsvorrichtung (40), die im Gebrauch dazu dient, um eine Gelschicht (41) aufzutragen, und eine zweite Fixierlösungsbeschichtungsvorrichtung (50) bereitgestellt sind, die im Gebrauch dazu dient, um eine zweite Fixierlösungsschicht (51) aufzutragen,
wobei die Gelbeschichtungsvorrichtung (40) nach der ersten Fixierlösungsbeschichtungsvorrichtung (30) angeordnet ist, und wobei die zweite Fixierlösungsbeschichtungsvorrichtung (50) nach der Gelbeschichtungsvorrichtung (40) angeordnet ist,
wobei die Gelbeschichtungsvorrichtung (40) zur Bereitstellung eines filmbildenden Materials konfiguriert ist, welches vorzugsweise Alginsäure und Salzverbindungen der Alginsäure (Alginate) beinhaltet, und
wobei die erste und die zweite Fixierlösung, die zur Auftragung mittels der ersten Fixiertosungsbeschichtungsvorrichtung (30) und der zweiten Fixierlösungsbeschichtungsvorrichtung (50) vorgesehen sind, Lösungen zum Aushärten und Fixieren des wasserlöslichen Filmbildungsmittels der Gelschicht (41) beinhalten, um einen unlöslichen Gelmembranfilm auszubilden.

2. Apparatur nach Anspruch 1, bei welcher eine Oberstoffeingabevorrichtung (80) zur Bereitstellung einer oberen Stofflage (81) zwischen der Gelbeschichtungsvorrichtung (40) und der zweiten Fixierlösungsbeschichtungsvorrichtung (50) angeordnet ist, sodass die Oberstoffeingabevorrichtung (80) im Gebrauch geeignet ist, um zuerst die obere Stofflage (81) entsprechend über der Gelschicht (41) anzubringen, wobei die obere Stofflage (81) zur Beschichtung durch die zweite Fixierlösungsschicht (51) geeignet ist.

3. Apparatur nach Anspruch 1 oder Anspruch 2, bei welcher die erste Fixierlösungsbeschichtungsvorrichtung (30) geeignet ist, um im Gebrauch die erste Fixierlösungsschicht (31) durch Sprühbeschichtung, Tauchbeschichtung oder eine Kombination daraus aufzutragen.

4. Apparatur nach Anspruch 1 oder Anspruch 2, bei welcher die zweite Fixierlösungsbeschichtungsvorrichtung (50) geeignet ist, um im Gebrauch die zweite Fixierlösungsschicht (51) durch Sprühbeschichtung, Tauchbeschichtung oder eine Kombination daraus aufzutragen.

5. Apparatur nach Anspruch 1 oder Anspruch 2, bei welcher die Materialien für die erste Fixierlösungsschicht (31) und die zweite Fixierlösungsschicht (51) nur Lösungen zum Aushärten und Fixieren umfassen, welche vorzugsweise Salzverbindungen beinhalten, die mit dem wasserlöslichen Filmbildungsmittel der Gelschicht (41) reaktionsfähig sind, oder eine Kombination der Lösung zum Aushärten und Fixieren mit speziellen Inhaltsstoffen zur Bereitstellung von bestimmten Funktionen für die Feuchtigkeitsfilme (100, 110) umfassen.

6. Apparatur nach Anspruch 1 oder Anspruch 2, bei welcher ein Vernetzungskontrollbereich (60) nach der zweiten Fixierlösungsbeschichtungsvorrichtung (50) vorgesehen ist, der im Gebrauch dazu dient, um wenigstens eine Vernetzungskontrolllösung (61) durch Sprühbeschichtung oder Tauchbeschichtung in dem Vernetzungskontrollbereich (60) zu verteilen, um die Vernetzungsreaktion in dem sich allmählich auf der unteren Stofflage (20) bildenden Feuchtigkeitsfilm (100, 110) zu steuern, sodass die Vernetzung zwischen der ersten Fixierlösungsschicht (31) und der Gelschicht (41) und/oder die Vernetzung zwischen der zweiten Fixierlösungsschicht (51) und der Gelschicht (41) ausgewaschen oder angehalten wird.

7. Apparatur nach Anspruch 6, bei welcher die Vernetzungskontrollläsung (61), die in dem Vernetzungskontrollbereich (60) verteilt wird, Reinwasser, Vernetzungsabbruchlösung, Reinwasser mit speziellen Inhaltsstoffen zur Bereitstellung von bestimmten Funktionen für die Feuchtigkeitsfilme (100, 110) und Vernetzungsabbruchlösung mit speziellen Inhaltsstoffen zur Bereitstellung von bestimmten Funktionen für die Feuchtigkeitsfilme (100, 110) beinhaltet.

8. Apparatur nach Anspruch 2, bei welcher die obere Stofflage (81), die durch die Oberstoffeingabevorrichtung (80) bereitgestellt wird, ein fortlaufender langer Stoffstreifen ist, der kontinuierlich bereitgestellt und korrespondierend über der Gelschicht (41) angebracht wird.

9. Apparatur nach Anspruch 1 oder Anspruch 2, bei welcher die untere Stofflage (20) ein fortlaufender langer Stoffstreifen ist, der durch das Eingangsende (12) der umlaufenden Beladungsfläche (11) in die umlaufende Beladungsfläche eintritt, glatt an der Beladungsfläche angebracht ist und synchron mit der Beladungsfläche (11) bewegt wird.

10. Apparatur nach Anspruch 1 oder Anspruch 2, bei welcher die untere Stofflage (20) an der umlaufenden Beladungsfläche (11) des Bandförderers (10) befestigt ist, sodass die untere Stofflage (20) synchron und kreisförmig mit dem Bandförderer (10) gedreht wird.

11. Apparatur nach Anspruch 10, bei welcher ein Schabe- und Reinigungswerkzeug (90) an einer Oberfläche der sich synchron und kreisförmig mit dem Bandförderer (10) drehenden unteren Stofflage (20) angeordnet ist, wobei das Schabe- und Reinigungswerkzeug (90) dazu dient, um nach der Bildung des Feuchtigkeitsfilms (100, 110) Reste der Fixierlösung (31, 51) oder des Gels (41) von der umlaufenden Beladungsfläche (11) oder der unteren Stofflage (20) abzukratzen und diese zu reinigen.

12. Verfahren zur kontinuierlichen Herstellung von Feuchtigkeitsfilmen, das mittels der Apparatur nach einem der von Anspruch 1 bis Anspruch 11 reichenden vorhergehenden Ansprüche durchgeführt wird, welches als Schritte umfasst:
Schritt 1:
Bereitstellen von wenigstens einer umlaufenden Beladungsfläche (11), welche als eine Arbeitsplattform zur Filmbildung verwendet wird, wobei die Beladungsfläche (11) eine nach oben weisende flache Oberfläche beinhaltet und entlang einer Förderrichtung von einem Eingangsende (12) zu einem Ausgangsende (13) rundumlaufend bewegt wird,
Schritt 2:
Bereitstellen einer unteren Stofflage (20) mit einer Wasseraufnahmefähigkeit, welche synchron mit der Beladungsfläche (11) bewegt wird, um der Reihe nach verschiedene filmbildende Materialien (31, 41, 51) aufzunehmen und einen Filmbildungsvorgang durchzuführen,
Schritt 3:
Aufbringen einer ersten Fixierlösungsschicht (31) auf die untere Stofflage (20) mittels einer ersten Fixierlösungsbeschichtungsvorrichtung (30) durch Sprühbeschichtung oder Tauchbeschichtung während die untere Stofflage (20) synchron mit der Beladungsfläche (11) bewegt wird,
Schritt 4:
Aufbringen einer Gelschicht (41) auf die erste Fixierlösungsschicht (31) mittels der Gelbeschichtungsvorrichtung (40) während die untere Stofflage (20) synchron mit der Beladungsfläche (11) zu einer Arbeitszone der Gelbeschichtungsvorrichtung (40) zurückbewegt wird und Vernetzungs- und Aushärtungsreaktionen zwischen der Gelschicht (41) und der ersten Fixierlösungsschicht (31) erfolgen, und
Schritt 5:
Aufbringen einer zweiten Fixierlösungsschicht (51) auf die Gelschicht (41) mittels der zweiten Fixierlösungsbeschichtungsvorrichtung (50) durch Sprühbeschichtung oder Tauchbeschichtung während die Gelschicht (41), die über der ersten Fixierlösungsschicht (31) aufgetragen ist, synchron mit der Beladungsfläche (11) zu einer Arbeitszone der zweiten Fixierlösungsbeschichtungsvorrichtung (50) zurückbewegt wird, wobei durch die Vernetzungs- und Aushärtungsreaktionen zwischen der zweiten Fixierlösungsschicht (51) und der Gelschicht (41) allmählich ein Feuchtigkeitsfilm (100) mit einer bestimmten Dicke an einer oberen Oberfläche der synchron mit der Beladungsfläche bewegten unteren Stofflage (20) gebildet wird und eine kontinuierliche Herstellung von Feuchtigkeitsfilmen (100) ohne Träger abgeschlossen wird.

13. Verfahren nach Anspruch 12, wobei das Verfahren nach dem Schritt 5 ferner einen Schritt 6 umfasst, um einen Vernetzungskontrollbereich (60) bereitzustellen, der die Vernetzungsreaktion in dem allmählich auf der unteren Stofflage (20) gebildeten Feuchtigkeitsfilm (100) durch wenigstens eine Vernetzungsabbruchlösung, welche durch Sprühbeschichtung oder Tauchbeschichtung in dem Vernetzungskontrollbereich (60) verteilt wird, kontrolliert, wobei die Vernetzungsreaktion zwischen der ersten Fixierlösungsschicht (31) und der Gelschicht (41) und die Vernetzungsreaktion zwischen der zweiten Fixierlösungsschicht (51) und der Gelschicht (41) angehalten oder ausgewaschen werden.

14. Verfahren zur kontinuierlichen Herstellung von Feuchtigkeitsfilmen, das mittels der Apparatur nach einem der von Anspruch 1 bis Anspruch 11 reichenden vorhergehenden Ansprüche durchgeführt wird, welches als Schritte umfasst:
Schritt 1:
Bereitstellen von wenigstens einer umlaufenden Beladungsfläche (11), welche als eine Arbeitsplattform zur Filmbildung verwendet wird, wobei die Beladungsfläche eine nach oben weisende flache Oberfläche beinhaltet und entlang einer Förderrichtung von einem Eingangsende (12) zu einem Ausgangsende (13) rundumlaufend bewegt wird,
Schritt 2:
Bereitstellen einer unteren Stofflage (20) mit einer Wasseraufnahmefähigkeit, welche synchron mit der Beladungsfläche (11) bewegt wird, um der Reihe nach verschiedene filmbildende Materialien (31, 41, 51) aufzunehmen und einen Filmbildungsvorgang durchzuführen,
Schritt 3:
Aufbringen einer ersten Fixierlösungsschicht (31) auf die untere Stofflage (20) mittels der ersten Fixierlösungsbeschichtungsvorrichtung (30) durch Sprühbeschichtung oder Tauchbeschichtung,
Schritt 4:
Aufbringen einer Gelschicht (41) auf die erste Fixierlösungsschicht (31) mittels der Gelbeschichtungsvorrichtung (40), wobei Vernetzungs- und Aushärtungsreaktionen zwischen der Gelschicht (41) und der ersten Fixierlösungsschicht (31) erfolgen, und
Schritt 5:
Bereitstellen einer oberen Stofflage (81), welche korrespondierend über der Gelschicht (41) angebracht wird,
Schritt 6:
Aufbringen einer zweiten Fixierlösungsschicht (51) auf die obere Stofflage (81) durch Sprühbeschichtung oder Tauchbeschichtung mittels der zweiten Fixierlösungsbeschichtungsvorrichtung (50), um Vernetzungs- und Aushärtungsreaktionen zwischen der zweiten Fixierlösungsschicht (81) und der Gelschicht (41) zu initiieren, wodurch die obere Stofflage (81) zwischen der zweiten Fixierlösungsschicht (51) und der Gelschicht (41) aufgrund der Vernetzungsreaktion dazwischen festgeklemmt und eingebunden wird, wobei ein Feuchtigkeitsfilm (110) mit einer bestimmten Dicke, der die obere Stofflage (20) enthält, allmählich an einer oberen Oberfläche der synchron mit der Beladungsfläche bewegten unteren Stofflage gebildet wird und eine kontinuierliche Herstellung der Feuchtigkeitsfilme (110) ohne Träger abgeschlossen wird.

15. Verfahren nach Anspruch 14, wobei das Verfahren nach dem Schritt 6 ferner einen Schritt 7 umfasst, um einen Vernetzungskontrollbereich (60) bereitzustellen, der die Vernetzungsreaktion in dem allmählich auf der unteren Stofflage (20) gebildeten Feuchtigkeitsfilm (110) durch wenigstens eine Vernetzungsabbruchlösung, welche durch Sprühbeschichtung oder Tauchbeschichtung in dem Vernetzungskontrollbereich (60) verteilt wird, kontrolliert, wobei die Vernetzungsreaktion zwischen der ersten Fixierlösungsschicht (31) und der Gelschicht (41) und die Vernetzungsreaktion zwischen der zweiten Fixierlösungsschicht (51) und der Gelschicht (41) angehalten oder ausgewaschen werden.

## Revendications

1. Appareil approprié pour fabriquer, en continu, des films (100), comprenant:
au moins un transporteur à courroie (10), une couche de tissu inférieure (20), et
des dispositifs d'enduction (30, 40, 50),
**caractérisé en ce que**
lesdits dispositifs d'enduction comprennent trois dispositifs d'enduction (30, 40, 50) appropriés pour, durant l'utilisation, enduire des premier, deuxième et troisième composés (31, 41, 51) tour à tour sur la couche de tissu inférieure (20) et
ledit transporteur à courroie (10) inclut au moins une surface de chargement circulante (11) utilisée en tant que plateforme de travail pour former le film (100, 110) et la surface de chargement circulante (11) inclut une surface plate tournée vers le haut et est appropriée pour, durant l'utilisation, un mouvement circulaire le long d'une direction de transport d'une extrémité d'entrée (12) à une extrémité de sortie (13) de celle-ci;
ladite surface de chargement est adaptée pour disposer la couche de tissu inférieure (20) sur la surface de la surface de chargement (11) et, durant l'utilisation, peut être déplacée de façon synchrone avec la surface de chargement (11) afin de recevoir divers matériaux filmogènes (31, 41, 51) tour à tour durant le mouvement de la couche de tissu inférieure (20) pour former, progressivement et en continu, le film (100, 110);
dans lequel les trois dispositifs d'enduction (30, 40, 50) sont agencés sur ou devant la surface de chargement (11) tour à tour d'une extrémité d'entrée (12) à une extrémité de sortie (13) le long d'une direction de transport de la surface de chargement (11);
et **en ce que** le film est un film hydratant (100) pour masques hydratants faciaux cosmétiques;
la couche de tissu inférieure (20) présente une hydrophilie;
les dispositifs d'enduction sont prévus sous forme de premier dispositif d'enduction de solution de fixage (30) approprié pour, durant l'utilisation, enduire une première couche de solution de fixage (31), un dispositif d'enduction de gel (40) approprié pour, durant l'utilisation, enduire une couche de gel (41), et un second dispositif d'enduction de solution de fixage (50) approprié pour, durant l'utilisation, enduire une seconde couche de solution de fixage (51);
dans lequel le dispositif d'enduction de gel (40) est disposé après le premier dispositif d'enduction de solution de fixage (30) et le second dispositif d'enduction de solution de fixage (50) est disposé après le dispositif d'enduction de gel (40);
dans lequel le dispositif d'enduction de gel (40) est configuré pour fournir un matériau filmogène, incluant de préférence de l'acide alginique et des composés de sel d'acide alginique (alginate); et
dans lequel les première et seconde solutions de fixage destinées à être appliquées par le premier dispositif d'enduction de solution de fixage (30) et le second dispositif d'enduction de solution de fixage (50) incluent des solutions pour durcir et fixer l'agent filmogène hydrosoluble de la couche de gel (41) pour former un film membranaire de gel insoluble.

2. Appareil selon la revendication 1, dans lequel un dispositif d'entrée de tissu supérieur (80) pour fournir une couche de tissu supérieure (81) est disposé entre le dispositif d'enduction de gel (40) et le second dispositif d'enduction de solution de fixage (50), pour que le dispositif d'entrée de tissu supérieur (80) soit approprié, durant l'utilisation, pour attacher premièrement la couche de tissu supérieure (81) par-dessus la couche de gel (41) de façon correspondante, dans lequel la couche de tissu supérieure (81) est appropriée pour être enduite par la seconde couche de solution de fixage (51).

3. Appareil selon la revendication 1 ou la revendication 2, dans lequel le premier dispositif d'enduction de solution de fixage (30) est approprié pour, durant l'utilisation, enduire la première couche de solution de fixage (31) par enduction par pulvérisation, enduction par immersion, ou leur association.

4. Appareil selon la revendication 1 ou la revendication 2, dans lequel le second dispositif d'enduction de solution de fixage (50) est approprié pour, durant l'utilisation, enduire la seconde couche de solution de fixage (51) par enduction par pulvérisation, enduction par immersion, ou leur association.

5. Appareil selon la revendication 1 ou la revendication 2, dans lequel des matériaux pour la première couche de solution de fixage (31) et la seconde couche de solution de fixage (51) incluent seulement des solutions pour durcir et fixer, de préférence incluant des composés de sel réactifs avec l'agent filmogène hydrosoluble de la couche de gel (41), ou incluent une association de la solution pour durcir et fixer avec des ingrédients spécifiques pour fournir des fonctions spécifiques aux films hydratants (100, 110).

6. Appareil selon la revendication 1 ou la revendication 2, dans lequel une zone de contrôle de réticulation (60) est disposée après le second dispositif d'enduction de solution de fixage (50) et est appropriée pour, durant l'utilisation, distribuer au moins une solution de contrôle de réticulation (61) par enduction par pulvérisation ou enduction par immersion dans la zone de contrôle de réticulation (60) pour contrôler la réaction de réticulation dans le film hydratant (100, 110) progressivement formé sur la couche de tissu inférieure (20); pour que la réticulation entre la première couche de solution de fixage (31) et la couche de gel (41) et/ou la réticulation entre la seconde couche de solution de fixage (51) et la couche de gel (41) soient éliminées par rinçage ou arrêtées.

7. Appareil selon la revendication 6, dans lequel la solution de contrôle de réticulation (61) distribuée dans la zone de contrôle de réticulation (60) inclut de l'eau pure, une solution d'arrêt de réticulation, de l'eau pure avec des ingrédients spécifiques fournissant des fonctions spécifiques aux films hydratants (100, 110) et une solution d'arrêt de réticulation avec des ingrédients spécifiques fournissant des fonctions spécifiques aux films hydratants (100, 110).

8. Appareil selon la revendication 2, dans lequel la couche de tissu supérieure (81) fournie par le dispositif d'entrée de tissu supérieur (80) est une longue bande continue de tissu fournie en continu et attachée par-dessus la couche de gel (41) de façon correspondante.

9. Appareil selon la revendication 1 ou la revendication 2, dans lequel la couche de tissu inférieure (20) est une longue bande continue de tissu entrée dans la surface de chargement circulante à travers l'extrémité d'entrée (12) de la surface de chargement circulante (11), attachée à la surface de chargement uniformément et déplacée de façon synchrone avec la surface de chargement (11).

10. Appareil selon la revendication 1 ou la revendication 2, dans lequel la couche de tissu inférieure (20) est fixée sur la surface de chargement circulante (11) du transporteur à courroie (10) pour que la couche de tissu inférieure (20) soit mise en rotation de façon synchrone et de façon circulaire avec le transporteur à courroie (10).

11. Appareil selon la revendication 10, dans lequel un outil de raclage et de nettoyage (90) est agencé sur une surface de la couche de tissu inférieure (20) mise en rotation de façon synchrone et de façon circulaire avec le transporteur à courroie (10) et l'outil de raclage et de nettoyage (90) est utilisé pour racler et nettoyer la solution de fixage (31, 51) ou le gel (41) résiduel de la surface de chargement circulante (11) ou la couche de tissu inférieure (20) après la formation du film hydratant (100, 110).

12. Procédé pour fabriquer, en continu, des films hydratants, réalisé par l'appareil selon l'une des revendications de la revendication 1 à la revendication 11, comprenant les étapes de:
Étape 1:
la fourniture d'au moins une surface de chargement circulante (11) utilisée en tant que plateforme de travail pour la formation de film et la surface de chargement (11) incluant une surface plate tournée vers le haut et déplacée de façon circulaire le long d'une direction de transport d'une extrémité d'entrée (20) à une extrémité de sortie (13);
Étape 2 :
la fourniture d'une couche de tissu inférieure (20) avec hydrophilie, qui est déplacée de façon synchrone avec la surface de chargement (11) pour recevoir divers matériaux filmogènes (31, 41, 51) tour à tour et réaliser une opération filmogène;
Étape 3:
l'enduction d'une première couche de solution de fixage (31) par-dessus la couche de tissu inférieure (20) par un premier dispositif d'enduction de solution de fixage (30) par enduction par pulvérisation ou enduction par immersion alors que la couche de tissu inférieure (20) est déplacée de façon synchrone avec la surface de chargement (11);
Étape 4:
l'enduction d'une couche de gel (41) par-dessus la première couche de solution de fixage (31) par le dispositif d'enduction de gel (40) alors que la couche de tissu inférieure (20) est déplacée vers l'arrière, de façon synchrone avec la surface de chargement (11), jusqu'à une distance de travail du dispositif d'enduction de gel (40) et présentant des réactions de réticulation ou de durcissement entre la couche de gel (41) et la première couche de solution de fixage (31); et
Étape 5:
l'enduction d'une seconde couche de solution de fixage (51) la couche de gel (41) par le second dispositif d'enduction de solution de fixage (50) par enduction par pulvérisation ou enduction par immersion alors que la couche de gel (41) enduite par-dessus la première couche de solution de fixage (31) est déplacée vers l'arrière de façon synchrone avec la surface de chargement (11) jusqu'à une distance de travail du second dispositif d'enduction de solution de fixage (50), dans lequel, par les réactions de réticulation ou de durcissement entre la seconde couche de solution de fixage (51) et la couche de gel (41), un film hydratant (100) avec une certaine épaisseur est progressivement formé sur une surface supérieure de la couche de tissu inférieure (20) qui est déplacée de façon synchrone avec la surface de chargement et une fabrication continue des films hydratants (100) sans supports est achevée.

13. Procédé selon la revendication 12, dans lequel, après l'étape 5, le procédé inclut en outre une étape 6 de la fourniture d'une zone de contrôle de réticulation (60) qui contrôle la réaction de réticulation dans le film hydratant (100) progressivement formé sur la couche de tissu inférieure (20); par au moins une solution d'arrêt de réticulation qui est distribuée par enduction par pulvérisation ou enduction par immersion dans la zone de contrôle de réticulation (60), la réaction de réticulation entre la première couche de solution de fixage (31) et la couche de gel (41) et la réaction de réticulation entre la seconde couche de solution de fixage (51) et la couche de gel (41) sont arrêtées ou éliminées par rinçage.

14. Procédé pour fabriquer, en continu, des films hydratants, réalisé par l'appareil selon l'une des revendications de la revendication 1 à la revendication 11, comprenant les étapes de:
Étape 1 :
la fourniture d'au moins une surface de chargement circulante (11) utilisée en tant que plateforme de travail pour la formation de film et la surface de chargement incluant une surface plate tournée vers le haut et déplacée de façon circulaire le long d'une direction de transport d'une extrémité d'entrée (12) à une extrémité de sortie (13);
Étape 2:
la fourniture d'une couche de tissu inférieure (20) avec hydrophilie qui est déplacée de façon synchrone avec la surface de chargement (11) pour recevoir divers matériaux filmogènes (31, 41, 51) tour à tour et réaliser une opération filmogène;
Étape 3:
l'enduction d'une première couche de solution de fixage (31) par-dessus la couche de tissu inférieure (20) par le premier dispositif d'enduction de solution de fixage (30) au moyen d'enduction par pulvérisation ou d'enduction par immersion;
Étape 4:
l'enduction d'une couche de gel (41) par-dessus la première couche de solution de fixage (31) par le dispositif d'enduction de gel (40) et présentant des réactions de réticulation ou de durcissement entre la couche de gel (41) et la première couche de solution de fixage (31); et
Étape 5:
la fourniture d'une couche de tissu supérieure (81) attachée par-dessus la couche de gel (41) de façon correspondante;
Étape 6:
l'enduction d'une seconde couche de solution de fixage (51) par-dessus la couche de tissu supérieure (81) par enduction par pulvérisation ou enduction par immersion par le second dispositif d'enduction de solution de fixage (50) pour initier des réactions de réticulation et de durcissement entre la seconde couche de solution de fixage (81) et la couche de gel (41); ainsi la couche de tissu supérieure (81) est limitée et contenue entre la seconde couche de solution de fixage (51) et la couche de gel (41) en raison de la réaction de réticulation entre celles-ci; dans lequel un film hydratant (110) avec une certaine épaisseur et contenant la couche de tissu supérieure (20) est progressivement formé sur une surface supérieure de la couche de tissu inférieure qui est déplacée de façon synchrone avec la surface de chargement et une fabrication continue des films hydratants (110) avec des supports est achevée.

15. Procédé selon la revendication 14, dans lequel, après l'étape 6, le procédé inclut en outre une étape 7 de la fourniture d'une zone de contrôle de réticulation (60) qui contrôle la réaction de réticulation dans le film hydratant (110) progressivement formé sur la couche de tissu inférieure (20); par au moins une solution d'arrêt de réticulation qui est distribuée par enduction par pulvérisation ou enduction par immersion dans la zone de contrôle de réticulation (60), la réaction de réticulation entre la première couche de solution de fixage (31) et la couche de gel (41) et la réaction de réticulation entre la seconde couche de solution de fixage (51) et la couche de gel (41) sont arrêtées ou éliminées par rinçage.
